# EUROPEAN PATENT APPLICATION

(11) **EP 3 835 408 A1**
(43) Date of publication of application: **16.06.2021**
(21) Application number: 19846227.7
(22) Date of filing: 05.07.2019
(51) Int. Cl.: C12M 1/20, C12M 1/22

(54) **CULTURE CHAMBER FOR MICROBIOLOGICAL COMPETITION ASSAYS USING VOLATILE COMPOUNDS**

(30) Priority: 10.08.2018 ES 201830817
(71) Applicant: Universidad de León, 24071 León (ES)
(72) Inventor: ÁLVAREZ GARCÍA, Samuel, 24071 León (ES); CASQUERO LUELMO, Pedro Antonio, 24071 León (ES); GUTIÉRREZ MARTÍN, Santiago, 24071 León (ES); MAYO PRIETO, Sara, 24071 León (ES); GONZÁLEZ LÓPEZ, Óscar, 24071 León (ES); CARRO HUERGA, Guzmán, 24071 24071 León (ES); SUÁREZ VILLANUEVA, Víctor, 24071 León (ES); RODRÍGUEZ GONZÁLEZ, Álvaro, 24071 León (ES)
(74) Representative: Clarke Modet & Co.
(86) International application number: PCT/ES2019/070475
(87) International publication number: WO 2020/030835

(57) **Abstract**

A culture chamber for microbiological competition assays using volatile compounds, comprising a first receptacle (1), a second receptacle (2) and a central part (3) between the first receptacle (1) and the second receptacle (2), wherein the receptacles (1, 2) comprise an outer wall (4) and a perimeter wall (5) perpendicular to the outer wall (4) and wherein the central part (3) comprises an intermediate wall (6) with at least one opening (13), and two side walls (7) that extend from the perimeter of the intermediate wall (6) in opposite directions, such that the side walls (7) of the central part (3) surround the perimeter walls (5) of the receptacles (1, 2) and the perimeter walls (5) of the receptacles (1, 2) are supported on the intermediate wall (6).

## Description

### Field of the invention

The present invention relates to a culture chamber for microbiological competition assays using volatile compounds. The culture chamber object of the invention is applicable in the field of microbiological analysis techniques, and more specifically that of culture plates or Petri dishes.

### Background of the invention

Culture plates, also known as Petri dishes, are generally shallow and circular receptacles of different sizes (with diameters that vary between 50 mm and 150 mm), although they can have other shapes and/or sizes.

Petri dishes are the fundamental element used in the culturing of microorganisms in a solid medium.

At present, the most common materials in the manufacture of these plates are plastic materials, although there are Petri dishes made of glass or other materials.

A Petri dish is made up of two basic elements: a base whereon the corresponding culture medium is located, and a lid which covers the preceding element.

There are various ways in which these two elements are adjusted or fitted, one being able to be kept loose with respect to the other or fixed therebetween by means of different anchoring elements (mechanical tightening or mechanical interlocking by rotation or hooking), avoiding in the latter case the contamination of cultures by accidental falls or opening. Moreover, the closure can be complete, thus generating a sealed space therein and preventing or limiting the exchange of gases with the outside of the plate (non-ventilated plates), or there may be several protrusions or projections in any of the two elements that create sufficient space therebetween so that there is a certain exchange or flow of gases between the inside and outside of the plate (ventilated plates).

For culturing microorganisms, it is of the utmost importance to manage gas flows and the applications thereof, including:
- providing the necessary oxygen concentration for aerobic microorganisms or limiting the same for anaerobic microorganisms;
- regulating the drying of the medium once it is deposited on the Petri dish, avoiding moisture condensation in the case of ventilated plates or preventing excess drying on long-term cultures on non-ventilated plates; and
- regulating the outlet to the outside of volatile compounds produced by the microorganisms in culture.

Competition assays between microorganisms have become essential tools for the *in vitro* characterisation of strains with capacity for biological control and for detecting bioactive compounds produced by the same. These experiments can be conducted in a solid medium (routinely in Petri dishes) or liquid medium (using other techniques and materials). In general, there are three types of *in vitro* competition assays between microorganisms in a solid medium:
- direct competition experiments, in which the strains of microorganisms come into direct contact;
- competition experiments using soluble compounds, in which strains of microorganisms only face soluble compounds produced by other strains without having direct physical contact therebetween; and
- competition experiments using volatiles, in which strains of microorganisms only face volatile compounds produced by other strains without having direct physical contact therebetween.

In the scientific literature, the following media for conducting this type of competition experiment using volatile compounds have been described:
- subdivided Petri dishes, which are plates with one or more partitions that divide the surface thereof into several portions where microorganisms can be cultured without coming into contact. These systems limit the growth area for each strain, preventing them from being cultured individually in the centre of the plate, and they are not feasible for measuring the radial growth of microorganism colonies. There is also a risk of cross-contamination, either through spores or because the mycelium is capable of exceeding the partition wall.
- introducing open Petri dishes into larger receptacles. This system is complex, involves an extra cost and makes handling and measuring during the period in which the experiment is conducted very difficult.
- arranging two opposing Petri dishes, wherein each of the strains is cultured in the centre of a Petri dish (routine) and later, after removing the lids thereof, the upper limits of both opposing plates are joined by some type of plastic adhesive. In this way, both cultures are opposite each other in a closed space (a type of chamber formed by the volume of the two plates), one growing in the bottom inner portion (generally the volatile-producing microorganism) and the other growing in the top inner portion of the chamber (generally the microorganism on which the effect of the compounds is evaluated). This is one of the most widely used systems for evaluating the activity of volatiles produced by biological control fungi. It allows for certain handling of the material once the experiment has started, taking measurements of the growth of the colonies and taking photographs of the same.

However, the option of arranging two opposing Petri dishes raises a series of problems, all derived from the lack of a specific commercial device designed to conduct this type of experiment:
- existence of cross-contamination: by spores, mycelium growth on the walls of the plates (leading to the contamination of the opposing culture) or by contact between the aerial parts of the mycelia. An average person skilled in microbiological analyses understands that this contamination distorts the results of the experiment. An intermediate membrane can be placed to prevent contamination; however, the arrangement thereof is complex and difficult to systematise since there are no specific anchoring or securing structures.
- difficulty in the opposing arrangement of the plates since there are no Petri dishes designed for this function, such that they do not fit properly and this hinders the handling and the very reproducibility of the experiment; in addition, there is no way to ensure that the fit was perfect or, conversely, that there were small spaces without contact between the edges of both plates.
- inability to regulate the inlet of air or exchange of gases with the outside; in these experiments with opposing plates, it is not possible to make use of these different arrangements since the lids must be discarded and the edges of the bases must be joined with some type of adhesive.
- unnecessary material waste by discarding the lids of the used plates due to lack of use in this type of experiment.

The present patent/utility model is aimed at solving the aforementioned problems generated in the design and development of competition experiments between microorganisms using volatile compounds. A novel culture chamber especially designed to conduct these experiments is described.

### Description of the invention

The object of the invention is a culture chamber for microbiological competition assays using volatile compounds comprising: a first receptacle, a second receptacle, and a central part between the first receptacle and the second receptacle.

The receptacles comprise an outer wall and a perimeter wall perpendicular to the outer wall, and the central part comprises an intermediate wall with at least one opening, and two side walls extending from the perimeter of the intermediate wall in opposite directions. In the plate object of the invention, the side walls of the central part surround the perimeter walls of the receptacles and the perimeter walls of the receptacles are supported on the intermediate wall.

In the culture chamber for microbiological competition assays using volatile compounds object of the invention, the intermediate wall of the central part comprises a first face opposite the first receptacle and a second face opposite the second receptacle, wherein the first face and the second face are smooth.

In another embodiment of the culture chamber for microbiological competition assays using volatile compounds object of the invention, the central part comprises an inner rim on at least one of the faces of the intermediate wall, such that the inner rim and the side wall form a housing for the end of the outer wall of a receptacle.

In another embodiment of the culture chamber for microbiological competition assays using volatile compounds object of the invention, the intermediate wall comprises a plurality of flanges on the first face and/or on the second face whereon the outer wall of a receptacle is supported.

In another embodiment, the culture chamber for microbiological competition assays using volatile compounds comprises a membrane or filter covering the opening in the intermediate wall of the central part, wherein the membrane or filter is porous, allowing the passage of air and volatile compounds and blocking the passage of larger elements.

The culture chamber for microbiological competition assays using volatile compounds object of the invention comprises a first rim located on the outer wall of the first receptacle and a second rim with a slightly different diameter located on the outer wall of the second receptacle, such that on the outer walls of a first receptacle and a second receptacle, the first rim fits into the second rim, preventing the relative movement between two stacked culture plates.

In another embodiment, the culture chamber for microbiological competition assays using volatile compounds object of the invention comprises at least one partition in at least one receptacle, such that the partition divides the inside of said receptacle into independent portions.

### Brief description of the drawings

As a complement to the description provided below, and for the purpose of helping to make the features of the invention more readily understandable, this specification is accompanied by a set of drawings based on which the innovations and advantages of the device object of the invention will be more easily understood.

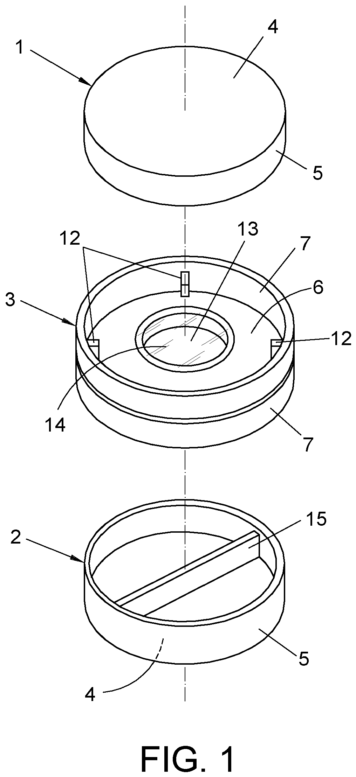

Figure 1 shows an exploded view of the culture chamber object of the invention.

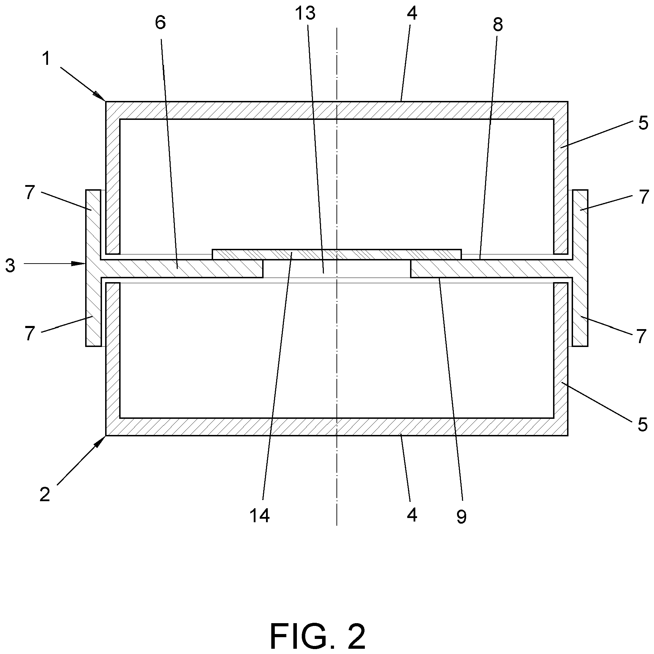

Figure 2 shows a cross-sectional view of the assembled culture chamber object of the invention.

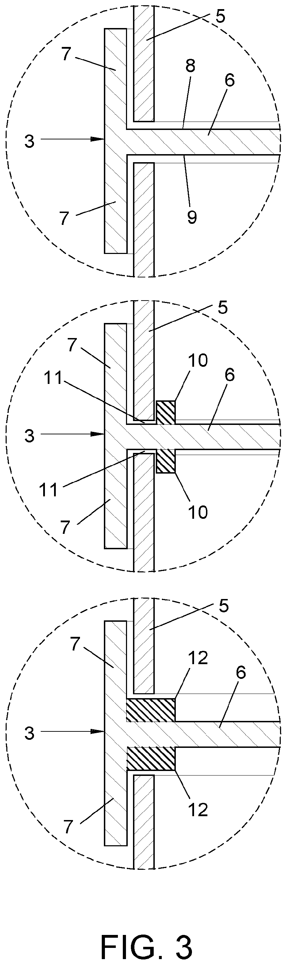

Figure 3 shows cross sections of two embodiments of the joining means between the two receptacles and the central part and a third cross section showing the flanges to provide a culture chamber with aeration.

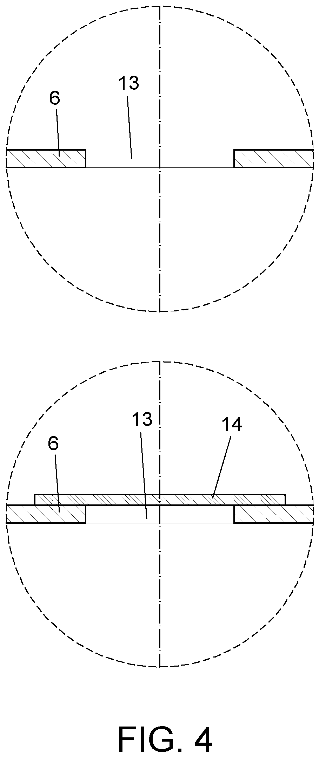

Figure 4 shows a detail view of the central opening in the horizontal intermediate wall of the intermediate element, with a variant in which said opening is open and another variant with said opening covered by a membrane or filter.

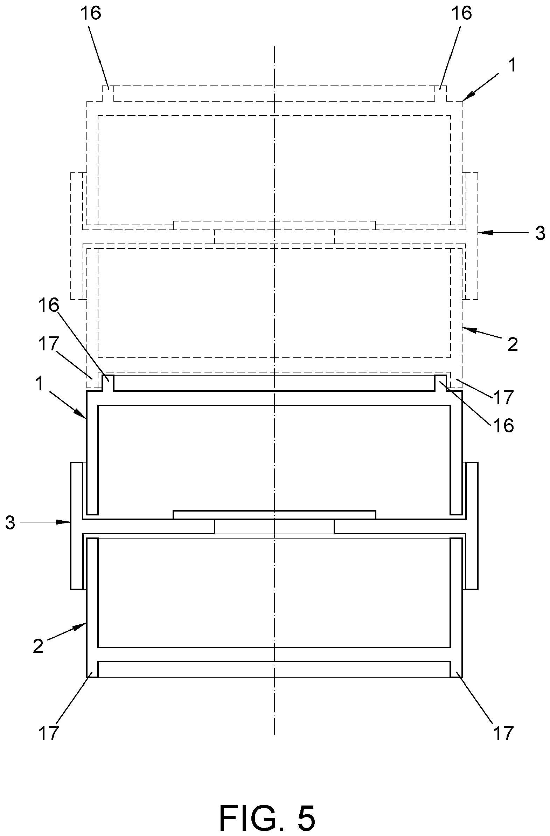

Figure 5 shows two stacked culture plates.

The different numerical references shown in the figures correspond to the following elements:
1. first receptacle,
2. second receptacle,
3. central part,
4. outer wall,
5. perimeter wall,
6. intermediate wall,
7. side wall,
8. first face,
9. second face,
10. inner rim,
11. housing,
12. flanges,
13. opening,
14. membrane or filter,
15. partition,
16. first rim, and
17. second rim.

### Detailed description of the invention

The culture chamber object of the invention comprises a first receptacle (1) and a second receptacle (2) and a central part (3) that is located between the first receptacle (1) and the second receptacle (2).

The receptacles (1, 2) in the preferred embodiment of the invention have an axisymmetric shape, and it is the element in which the nutrient medium for the culture is placed. The central part (3) in the preferred embodiment of the invention also has an axisymmetric shape and a diameter slightly greater than the diameter of the receptacles (1, 2).

The receptacles (1, 2) comprise an outer wall (4) which, in the preferred embodiment of the invention, is circular in shape and smooth on the inner face thereof, and also comprise a perimeter wall (5) that protrudes perpendicular to the outer wall (4).

The central part (3) comprises an intermediate wall (6) and two side walls (7) extending from the perimeter of the intermediate wall (6) in opposite directions. The intermediate wall (6) comprises a first face (8) towards the first receptacle (1) and a second face (9) towards the second receptacle (2).

To fix the central part (3) and the receptacles (1, 2), the central part (3) may comprise an inner rim (10) on at least one of the faces (8, 9) of the intermediate wall (6), such that the inner rim (10) and the side wall (7) form a housing (11) for the end of the perimeter wall (5) of a receptacle (1, 2), both elements thus becoming interlocked.

Furthermore, the intermediate wall (6) houses an opening (13), which is circular in the preferred embodiment of the invention. The diameter of the opening (13) in the preferred embodiment of the invention is approximately one third of the diameter of the intermediate wall (6). However, it is possible that instead of housing a single opening (13), the intermediate wall (6) houses more than one opening (13) that has shapes and sizes different from the one proposed in the preferred embodiment, while allowing the free exchange of gases between both receptacles (1, 2) of the culture chamber and limiting the possibilities of cross-contamination of the microorganisms in culture.

The opening (13) of the culture chamber object of the invention can be covered by a membrane or a filter (14) which is porous so that it allows the passage of air and volatile compounds, but blocks the passage of larger elements like fungal spores. These elements, membrane or filter (14), can be industrially adhered to the contour of the opening (13), to part or all of any of the faces (8, 9) of the intermediate wall (6) of the central part or they can also be fixed by mechanical means to any of the faces (8, 9) of the intermediate wall (6).

In the preferred embodiment of the invention, the dimensions of the perimeter wall (5) of both receptacles (1, 2) are identical, although there are alternative embodiments in which the dimensions of the perimeter wall (5) of each receptacle (1, 2) are different from each other.

In the preferred embodiment of the invention, the dimensions of the outer wall (4) of the receptacles (1, 2) are identical, although there are alternative embodiments in which the dimensions of the two outer walls (4) are different.

In the preferred embodiment of the invention, the dimensions of the two side walls (7) of the central part (3) are identical, although there are alternative embodiments in which the dimensions of the two side walls (7) are different.

The dimensions of the intermediate wall (6) of the central part (3) are slightly greater than the dimensions of the outer walls (4) of the receptacles (1, 2); considering that, in the preferred embodiment of the invention, said walls (4, 6) are circular, the diameter of the intermediate wall (6) is greater than the diameter of the outer walls (4). Likewise, in the preferred embodiment of the invention, the perimeter wall (5) of the receptacles (1, 2) has a greater height than the side walls (7) of the intermediate part.

In the culture chamber object of the invention, the central part (3) fits and rests on the second receptacle (2) while allowing the first receptacle (1) to fit and rest on it, such that the perimeter wall (5) of each receptacle (1, 2) fits into one of the side walls (7) of the central part (3); in addition, the perimeter walls (5) of the receptacles (1, 2) are supported on the intermediate wall (6) of the central part (3), such that the receptacles (1, 2) are opposite each other and create an inner chamber made up of the volume of both receptacles (1, 2) arranged symmetrically and partially communicated through the opening (13) in the intermediate wall (6) of the central part (3).

The fit between the central part (3) and the receptacles (1, 2) gives stability to the culture chamber as a whole and holds the components together when the culture chamber is not being handled.

The intermediate wall (6) of the central part (3) is parallel to the outer walls (4) of the receptacles (1, 2).

In the preferred embodiment of the invention, the present culture chamber is made of transparent plastic materials known in the manufacture of other culture plates. However, the culture chamber, in alternative embodiments, is made of glass or any other materials suitable for its function.

Although in the preferred embodiment of the invention the dimensions of both receptacles (1, 2) are identical, have an axisymmetric shape and are shallow, there are alternative embodiments in which the dimensions of the receptacles (1, 2) are not identical and the shape can vary, since both the receptacles (1, 2) and the central part (3) can be square, rectangular, etc.

In the preferred embodiment of the invention, both the first face (8) and the second face (9) of the intermediate wall (6) of the central part (3) are smooth and, therefore, continuous contact is generated with the end of the perimeter walls (5) of the receptacles (1, 2), thus limiting the exchange of gases with the outside of the culture chamber (non-ventilated plates). However, in alternative embodiments, the first face (8) and/or the second face (9) may have flanges (12) that prevent continuous contact and that, therefore, form gaps through which a certain exchange of gases with the outside occurs (ventilated plates). There are embodiments in which smooth faces and faces with flanges (12) are combined so that any of the two receptacles (1, 2) can be vented independently. This gives great versatility to the present invention, allowing the culture conditions to be varied and selecting those conditions that are most suitable for each specific experiment.

Although in the preferred embodiment of the invention the inner faces of both receptacles (1, 2) are smooth, in alternative embodiments one or more partitions (15) which subdivide the inside of one or both of receptacles (1, 2) into two or more independent portions may appear, allowing for several independent cultures in the same receptacle (1, 2).

The culture chamber object of the invention comprises stacking means, which make it possible to stack two or more culture plates. Said stacking means comprise a first rim (16) located on the outer wall (4) of the first receptacle (1) and a second rim (17) with a diameter slightly different from the first rim (16) located on the outer wall (4) of the second receptacle (2), such that opposite the outer walls (4) of a first receptacle (1) and a second receptacle (2), the first rim (16) fits into the second rim (17) and the relative movement between two stacked culture plates is prevented.

## Claims

1. A culture chamber for microbiological competition assays using volatile compounds, **characterised in that** it comprises:
- a first receptacle (1),
- a second receptacle (2), and
- a central part (3) between the first receptacle (1) and the second receptacle (2),
wherein the receptacles (1, 2) comprise an outer wall (4) and a perimeter wall (5) perpendicular to the outer wall (4), and wherein the central part (3) comprises an intermediate wall (6) with at least one opening (13), and two side walls (7) that extend from the perimeter of the intermediate wall (6) in opposite directions, such that the side walls (7) of the central part (3) surround the perimeter walls (5) of the receptacles (1, 2) and the perimeter walls (5) of the receptacles (1, 2) are supported on the intermediate wall (6) of the central part (3).

2. The culture chamber for microbiological competition assays using volatile compounds according to claim 1, **characterised in that** the intermediate wall (6) of the central part (3) comprises a first face (8) opposite the first receptacle (1) and a second face (9) opposite the second receptacle (2), wherein the first face (8) and the second face (9) are smooth.

3. The culture chamber for microbiological competition assays using volatile compounds according to claim 1, **characterised in that** the intermediate wall (6) of the central part (3) comprises a first face (8) opposite the first receptacle (1) and a second face (9) opposite the second receptacle (2), wherein at least one of the faces (8, 9) comprises a plurality of flanges (12) whereon the perimeter wall (5) of a receptacle (1, 2) is supported, thus facilitating the exchange of gases with the outside.

4. The culture chamber for microbiological competition assays using volatile compounds according to any of claims 1 to 3, **characterised in that** the central part (3) comprises an inner rim (10) on at least one of the faces (8, 9) of the intermediate wall (6), such that the inner rim (10) and the side wall (7) form a housing (11) for the end of the perimeter wall (5) of one or both receptacles (1, 2) thus becoming interlocked.

5. The culture chamber for microbiological competition assays using volatile compounds according to any of claims 1 to 4, **characterised in that** it comprises a membrane or filter (14) covering the opening (13) in the intermediate wall (6) of the central part (3), wherein the membrane or filter (14) is porous, allowing the passage of air and volatile compounds and blocking the passage of larger elements.

6. The culture chamber for microbiological competition assays using volatile compounds according to any of claims 1 to 5, **characterised in that** it comprises:
- a first rim (16) located on the outer wall (4) of the first receptacle (1), and
- a second rim (17) located on the outer wall (4) of the second receptacle (2),
wherein opposite the outer walls (4) of a first receptacle (1) and a second receptacle (2), the first rim (16) fits into the second rim (17), preventing the relative movement between two stacked culture plates.

7. The culture chamber for microbiological competition assays using volatile compounds according to any of claims 1 to 6, **characterised in that** it comprises at least one partition (15) in at least one receptacle (1, 2), such that the partition (15) divides the inside of said receptacle (1, 2) into independent portions.
